# EUROPEAN PATENT APPLICATION

(11) **EP 4 697 373 A1**
(43) Date of publication of application: **18.02.2026**
(21) Application number: 24788392.9
(22) Date of filing: 30.01.2024
(51) Int. Cl.: H01J 37/08, A61N 5/10, H05H 13/00

(54) **ION SOURCE, CIRCULAR ACCELERATOR, AND PARTICLE BEAM THERAPY SYSTEM**

(30) Priority: 14.04.2023 JP 2023066354
(71) Applicant: HITACHI HIGH-TECH CORPORATION, Tokyo 105-6409 (JP)
(72) Inventor: IWATA, Takeshi, Tokyo 100-8280 (JP); SEKI, Takayoshi, Tokyo 100-8280 (JP); ASANO, Hidehito, Tokyo 100-8280 (JP)
(74) Representative: MERH-IP Matias Erny Reichl Hoffmann Patentanwälte PartG mbB
(86) International application number: PCT/JP2024/002811
(87) International publication number: WO 2024/214362

(57) **Abstract**

A cathode 33, a cathode support tool 35 that supports the cathode 33 and applies and supplies a voltage, cooling tubes 24 and 25 that cool the cathode support tool 35, and a gas supply flow path 22, 22A, 22B, 22C, or 22D that supplies a gas to an electrical discharge chamber 36 are provided, and the cooling tubes 24 and 25 are disposed on a flow-path inner peripheral side of the gas supply flow path 22, 22A, 22B, 22C, or 22D. As a result, an ion source, a circular accelerator, and a particle beam therapy system that are capable of achieving both cooling and gas supply to an electrical discharge chamber with a compact configuration are provided.

## Description

### Technical Field

The present invention relates to an ion source suitably used for a circular accelerator, and further relates to a circular accelerator and a particle beam therapy system.

### Background Art

PTL 1 describes that in a cyclotron internal negative ion source including a tubular anode, an electrical discharge chamber formed by a space inside the anode, two cathodes disposed on both end sides of the electrical discharge chamber, and an ion extraction port formed in the anode for extracting ions from the electrical discharge chamber to the outside, a recess for a low electron temperature region is formed in an anode wall portion between the electrical discharge chamber and the ion extraction port.

### Citation List

### Patent Literature

PTL 1: JP 3426406 B2

### Summary of Invention

### Technical Problem

The ion source is an apparatus that generates plasma therein and emits a part of the generated plasma from an emission port as a charged beam, and is used in, for example, accelerators used for cancer treatment using proton beams, particle physics research, and the like.

In the ion source, a water cooling tube for cooling the electrical discharge chamber and a tube for supplying a sample gas as a raw material of extracted ions are connected from an external supply source. PTL 1 describes an example of such an ion source for a cyclotron.

For the connection of the water cooling tube and the gas tube in the ion source of the accelerator, it is necessary to form a hole in a magnetic pole at the time of insertion from a direction perpendicular to a plane of a radiofrequency acceleration electrode, and it is desirable to form such a hole as small as possible. In addition, it is necessary to provide insulation between a charged portion and the surrounding magnetic pole to avoid electrical discharge.

However, in PTL 1, since the water cooling tube and the gas supply tube are disposed independently, there are problems that a diameter of the ion source becomes large when the tubes are bundled together, and the hole of the magnetic pole becomes large due to each required insulation distance, which deteriorates manufacturability of the accelerator and makes miniaturization difficult.

An object of the present invention is to provide an ion source, a circular accelerator, and a particle beam therapy system that are capable of achieving both cooling and gas supply to an electrical discharge chamber with a compact configuration.

### Solution to Problem

The present invention includes a plurality of means for solving the above problems. As an example, an ion source includes a cathode; a cathode support tool that supports the cathode and applies and supplies a voltage; a cooling flow path that cools the cathode support tool; and a gas supply flow path that supplies a gas to an electrical discharge chamber, in which the cooling flow path is disposed on a flow-path inner peripheral side of the gas supply flow path.

### Advantageous Effects of Invention

According to the present invention, it is possible to achieve both cooling and gas supply to the electrical discharge chamber with a compact configuration. Problems, configurations, and effects other than those described above will become apparent by the following description of embodiments.

### Brief Description of Drawings

[FIG. 1] FIG. 1 is a diagram illustrating an overall configuration of a particle beam therapy system using a circular accelerator of the present invention.
[FIG. 2] FIG. 2 is a view illustrating a side cross section of the circular accelerator illustrated in FIG. 1.
[FIG. 3] FIG. 3 is a view illustrating a lateral cross section of the circular accelerator illustrated in FIG. 1.
[FIG. 4] FIG. 4 is a view schematically illustrating a side surface around an ion source of an embodiment.
[FIG. 5] FIG. 5 is a view schematically illustrating a side surface around an ion source according to a first modified example.
[FIG. 6] FIG. 6 is a view schematically illustrating a side surface around an ion source according to a second modified example.
[FIG. 7] FIG. 7 is a view schematically illustrating a side surface around an ion source according to a third modified example.
[FIG. 8] FIG. 8 is a view schematically illustrating a side surface around an ion source according to a fourth modified example.

### Description of Embodiments

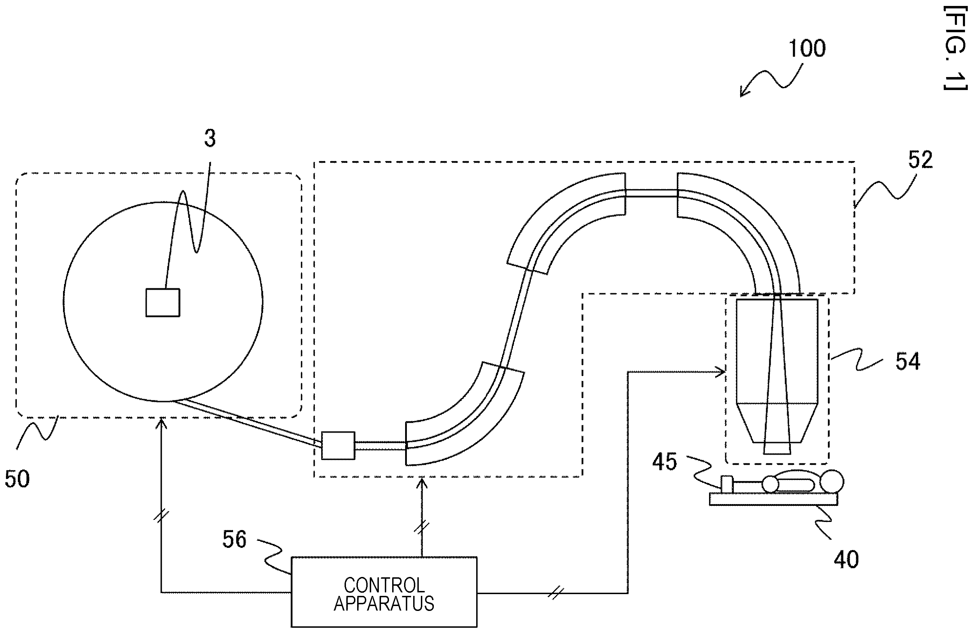

An embodiment of an ion source, a circular accelerator, and a particle beam therapy system of the present invention will be described with reference to FIGS. 1 to 8. The following embodiment is merely an example, and the present invention is not limited to the following specific aspects. The present invention itself can be modified into various forms other than the following embodiment.

Note that, in the drawings used in the present specification, the same or corresponding constituent elements are denoted by the same or similar reference signs, and a repeated description of the constituent elements may be omitted.

First, an overall configuration of the particle beam therapy system and a configuration of a related apparatus will be described with reference to FIG. 1. FIG. 1 is a diagram illustrating the overall configuration of the particle beam therapy system of the present embodiment.

A particle beam therapy system 100 illustrated in FIG. 1 includes a cyclotron circular accelerator 50, a beam transport line 52, an irradiation nozzle 54, a treatment couch 40, a control apparatus 56, and the like.

In the particle beam therapy system 100, ions generated by an ion source 3 are accelerated by the circular accelerator 50 to form an ion beam. The ion beam accelerated to a desired energy by the circular accelerator 50 is extracted from the circular accelerator 50 and transported to the irradiation nozzle 54 by the beam transport line 52. The transported ion beam is shaped to match a shape of a target volume by the irradiation nozzle 54, and a target on a patient 45 lying on the treatment couch 40 is irradiated with a predetermined amount of ion beam.

An operation of each apparatus and device in the particle beam therapy system 100 including the circular accelerator 50 is controlled by the control apparatus 56.

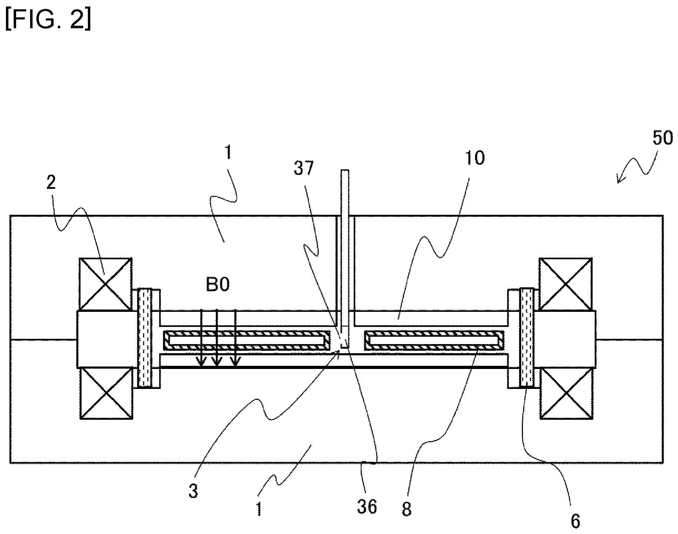

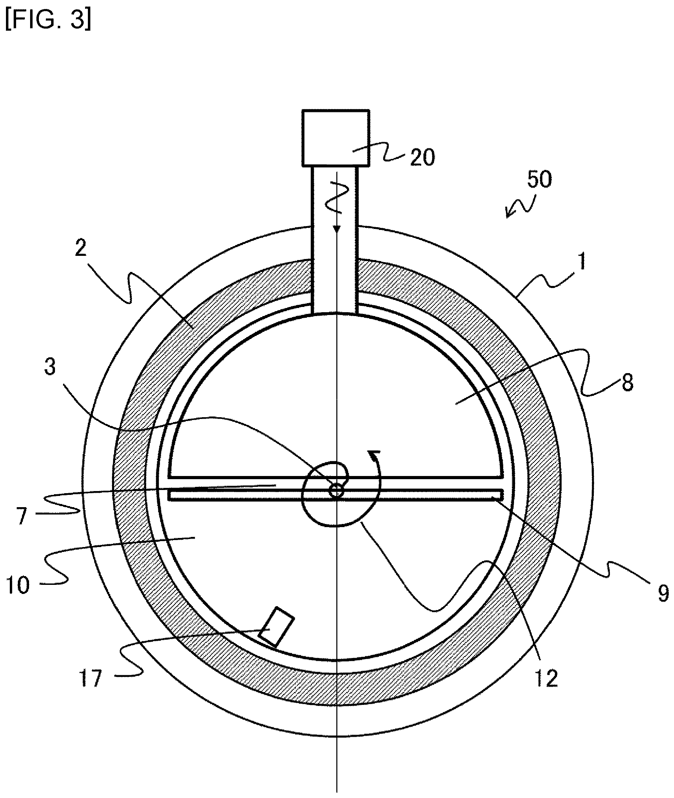

Next, a structure of the circular accelerator 50 will be described with reference to FIGS. 2 and 3. FIG. 2 is a cross-sectional view of a side surface of the accelerator of the present embodiment, and FIG. 3 is a lateral cross-sectional view.

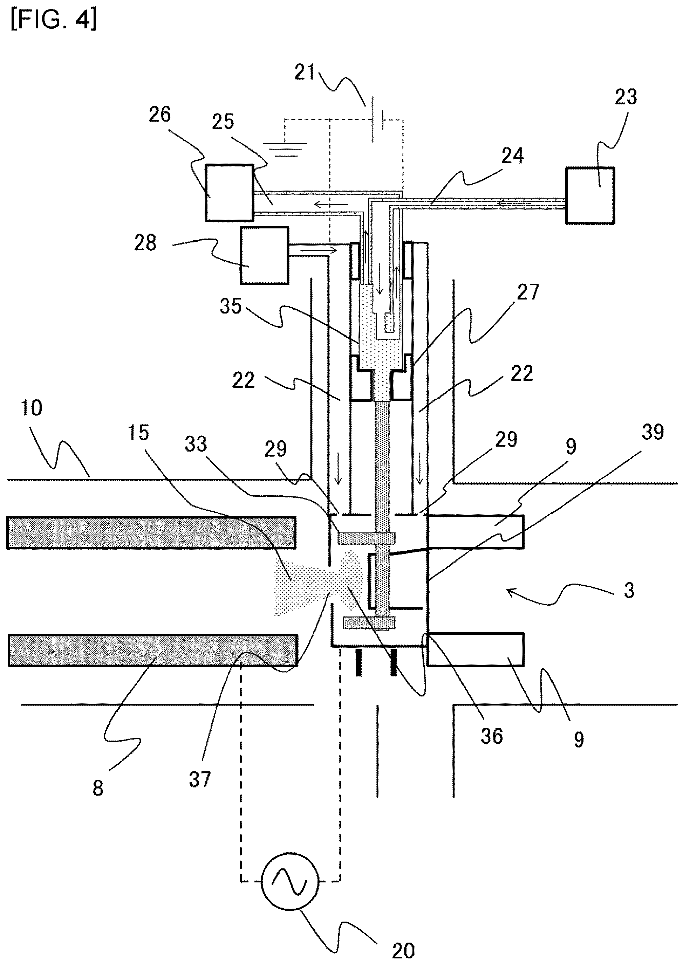

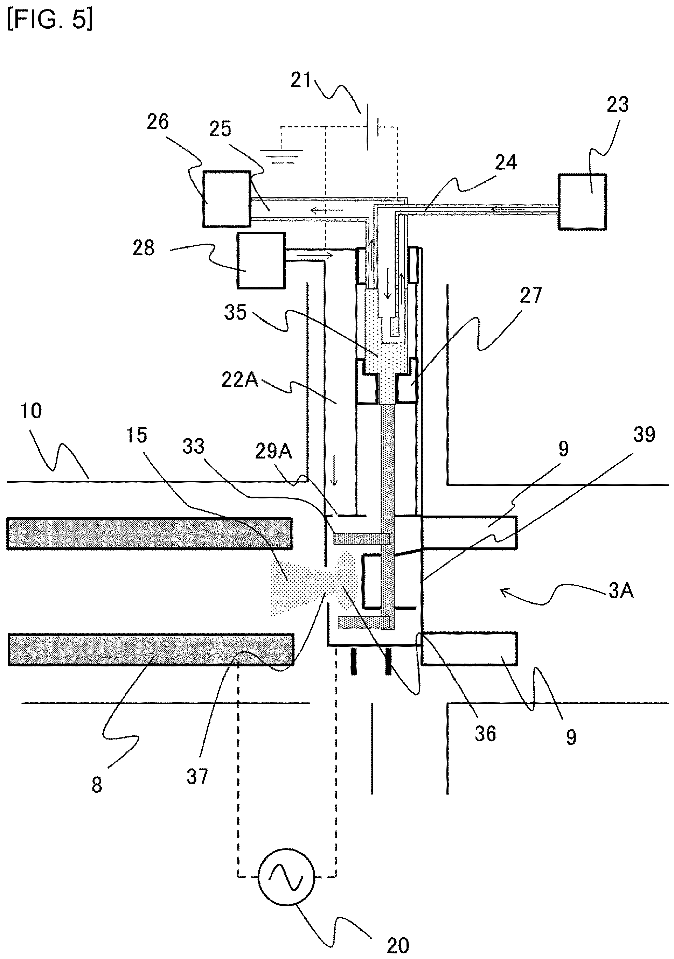

As illustrated in FIGS. 2 and 3, the cyclotron circular accelerator 50 includes main magnetic poles 1, an annular coil 2, a vacuum vessel 6, a radiofrequency acceleration electrode 8, and the ion source 3. The ion source is not limited to the form illustrated in FIG. 4 described below, and any one of ion sources 3A, 3B, 3C, and 3D illustrated in FIGS. 5 to 8 can be adopted.

The main magnetic poles 1 are a pair of magnetic bodies installed so as to face each other, and are made of, for example, iron. The main magnetic poles 1 are provided with a pair of upper and lower magnetic poles 10 facing each other so as to generate a closed orbit 12 of the beam, and generate a magnetic field between the magnetic poles 10.

For example, a magnetic field B0 as illustrated in FIG. 2 is generated by the magnetic poles 10, and a gradient magnetic field is formed outward from the center of circulation of the magnetic poles 10, so that a focusing force for the circulating ion beam is generated, thereby achieving stable circulation. A surface shape of each of facing surfaces with a magnetic pole gap of the pair of upper and lower magnetic poles 10 that generates the magnetic field B0 is a symmetrical shape. Alternatively, it is possible to form a magnetic pole to which a convergence effect is added by forming a magnetic pole shape provided with unevenness in a traveling direction of the beam.

The vacuum vessel 6 is sandwiched between the main magnetic poles 1, is formed as one vacuum vessel with the magnetic poles 10 as an inner surface, and forms a magnetic circuit. The vacuum vessel 6 is a non-magnetic body. A separate vacuum vessel in which the magnetic poles 10 do not serve as the inner surface of the vacuum vessel may be separately provided in the gap between the magnetic poles 10.

The annular coil 2 is installed closer to the atmosphere than the vacuum vessel 6, and generates the magnetic field B0 between the pair of upper and lower main magnetic poles 1. The annular coil 2 can similarly generate the magnetic field even in the case of a coil made of a normal conducting material or a coil made of a superconducting material. The annular coil 2 may be installed in the vacuum vessel 6, and an installation position of the annular coil 2 is not particularly limited.

The ion source 3 is disposed at an inner portion of the magnetic pole 10. Plasma is formed in the electrical discharge chamber 36 by electrons emitted from a cathode 33 in the electrical discharge chamber 36. A radiofrequency electric field is generated between a radiofrequency acceleration electrode 8 and the ion source 3 and between the ion source 3 and a ground electrode 9 having the same potential as the ion source 3 by a radio frequency supplied by a radiofrequency power supply 20. Due to the radiofrequency electric field, the ions are extracted from the plasma generated in the electrical discharge chamber 36 through an extraction hole 37, and an emitted beam 15 is formed.

The formed emitted beam 15 formed circulates while drawing the helical closed orbit 12 by the magnetic field B0 generated by the magnetic poles 10 and the electric field generated in an acceleration gap 7 between the radiofrequency acceleration electrode 8 and the ground electrode 9, is accelerated every time the emitted beam passes through the acceleration gap 7, is accelerated to a predetermined energy while increasing energy, and is then extracted to the outside of the main magnetic poles 1. An amount of the beam at this time is measured by a current monitor 17.

Next, details of the ion source 3 will be described with reference to FIG. 4. FIG. 4 is a view illustrating details of the ion source 3 of FIG. 2, and is a view in a case where the ion source 3 is disposed in a direction parallel to the magnetic field generated by the magnetic poles 10.

The ion source 3 illustrated in FIG. 4 is installed between the magnetic poles 10 facing each other, and the electrical discharge chamber 36 that generates the ions is disposed in parallel with a central axis of the circulating ion beam.

More specifically, the ion source 3 is a penning ionization gauge (PIG) type ion source 3 including the cathode 33 that generates the electrons, a cathode support tool 35 that supports the cathode 33 and applies and supplies a voltage to the cathode 33, an electrical discharge power supply 21 that applies an applied voltage for electrical discharge between the cathode 33 and an electrical discharge vessel 39 that serves as an anode, the electrical discharge chamber 36 that generates the ions by the applied voltage, the extraction hole 37 through which the ions are emitted from the electrical discharge chamber 36, the electrical discharge vessel 39, and the like.

The ion source 3 is further provided with a gas supply flow path 22 that supplies a sample gas to be supplied from a gas supply source 28 to the electrical discharge chamber 36, and cooling tubes 24 and 25 for cooling the cathode support tool 35 and the cathode 33.

In the ion source 3 of the present embodiment, the cooling tubes 24 and 25 and the gas supply flow path 22 have a triple structure in which the three tubes have a common central axis and have a coaxial structure, and the center corresponds to the cooling tube 24 on a cooling water supply side, the second axis from the center corresponds to the cooling tube 25 on a cooling water discharge side, and the outermost axis corresponds to the gas supply flow path 22.

A coolant supplied from a cooling material supply source 23, such as cooling water, is supplied to the cooling tube 24 to cool the cathode support tool 35 and cool the cathode 33 via the cathode support tool 35, and the coolant used for cooling is sent to a cooling material returning unit 26 via the cooling tube 25.

The cooling tubes 24 and 25 are not limited to a case where the cooling tube 24 on a coolant supply side is on an inner peripheral side, and the cooling tube 25 on a returning side is on an outer peripheral side. The cooling tube on the returning side may be on the inner peripheral side, and the cooling tube on the supply side may be on the outer peripheral side. In either case, the gas supply flow path 22 is positioned on an outer periphery of the cooling tube on the outer peripheral side.

As illustrated in FIG. 4, a cross-sectional area of a gas supply hole 29, which is an outlet of the gas supply flow path 22, is smaller than a cross-sectional area of the gas supply flow path 22.

Furthermore, the gas supply hole 29 is disposed vertically above the extraction hole 37 that emits the generated ions from the ion source 3. Since the ion source 3 is inserted from vertically above the magnetic poles 10, the gas supply hole 29 is disposed vertically above the extraction hole 37 that emits the generated ions from the ion source 3. However, in a case where the ion source 3 is inserted from vertically below the magnetic poles 10, the gas supply hole 29 may be disposed vertically below the extraction hole 37.

In the ion source 3, the electrical discharge power supply 21 applies a voltage between the cathode 33 and the electrical discharge vessel 39 electrically connected to the ground electrode 9 through the cooling tubes 24 and 25 and the cathode support tool 35. When the cathode 33 is set to a negative potential for the electrical discharge vessel 39, the electrons are emitted from the cathode 33 toward the electrical discharge chamber 36.

Since the cooling tubes 24 and 25 are at a high voltage and the gas supply flow path 22 is grounded, an insulating spacer 27 is provided between the cooling tubes 24 and 25 and the gas supply flow path 22 to form a gap between the cooling tubes 24 and 25 and the gas supply flow path 22, so that no electrical discharge occurs in the gas supply flow path 22.

In the coaxial structure as in the present embodiment, since an electric field between the cooling tubes 24 and 25 on a high-voltage side and the gas supply flow path 22 on a ground side becomes uniform, an insulation distance can be shortened, and a piping system of the ion source 3 can be downsized.

A hole penetrating through the main magnetic pole 1 and the magnetic pole 10 is provided, and the ion source 3 is inserted from an upper portion of the main magnetic pole 1. Here, as the holes are provided in the main magnetic pole 1 and the magnetic pole 10, distribution of the magnetic field B0 generated between the magnetic poles 10 is affected and changed. As a diameter of the hole is smaller, the influence on the magnetic field B0 is smaller, and correction can be easily performed by installing the shape of the magnetic pole 10 or a separate iron piece.

Therefore, by adopting the coaxial structure, the holes formed in the main magnetic pole 1 and the magnetic pole 10 can be reduced, and a design of the accelerator can be facilitated.

The cooling tubes 24 and 25 and the gas supply flow path 22 are made of a conductive material, and stainless steel is preferable, but the material of cooling tubes 24 and 25 and the gas supply flow path 22 is not particularly limited. A material of the insulating spacer 27 is not particularly limited as long as the material is an electrically insulating material such as alumina or fiber reinforced plastic (FRP).

In the present embodiment, an example in which water as the coolant flows through the cooling tubes 24 and 25 has been described, but air cooling, oil cooling, or the like may be used, and there is no particular limitation.

Hereinafter, modified examples of the ion source will be described with reference to FIGS. 5 to 8.

FIG. 5 illustrates an ion source 3A according to a first modified example. The ion source 3A illustrated in FIG. 5 is different from the ion source 3 illustrated in FIG. 4 in that central axes of cooling tubes 24 and 25 are not coaxial with a central axis of a gas supply flow path 22A, but are offset from the central axis of the gas supply flow path 22A.

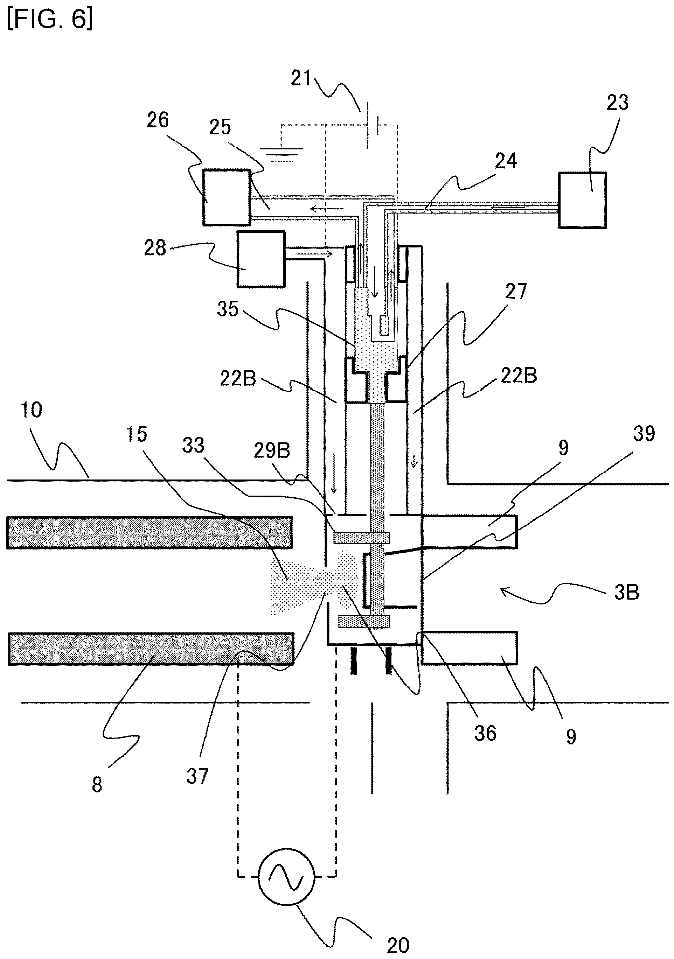

FIG. 6 illustrates an ion source 3B according to a second modified example. The ion source 3B illustrated in FIG. 6 is different from the ion source 3 illustrated in FIG. 4 in that a gas supply hole 29B for supplying a gas from a gas supply flow path 22B to an electrical discharge chamber 36 is disposed only on a side adjacent to an extraction hole 37. In such an ion source 3B, since an amount of gas supplied to the electrical discharge chamber 36 can be reduced, the gas can be appropriately supplied to the electrical discharge chamber 36 in which plasma is generated.

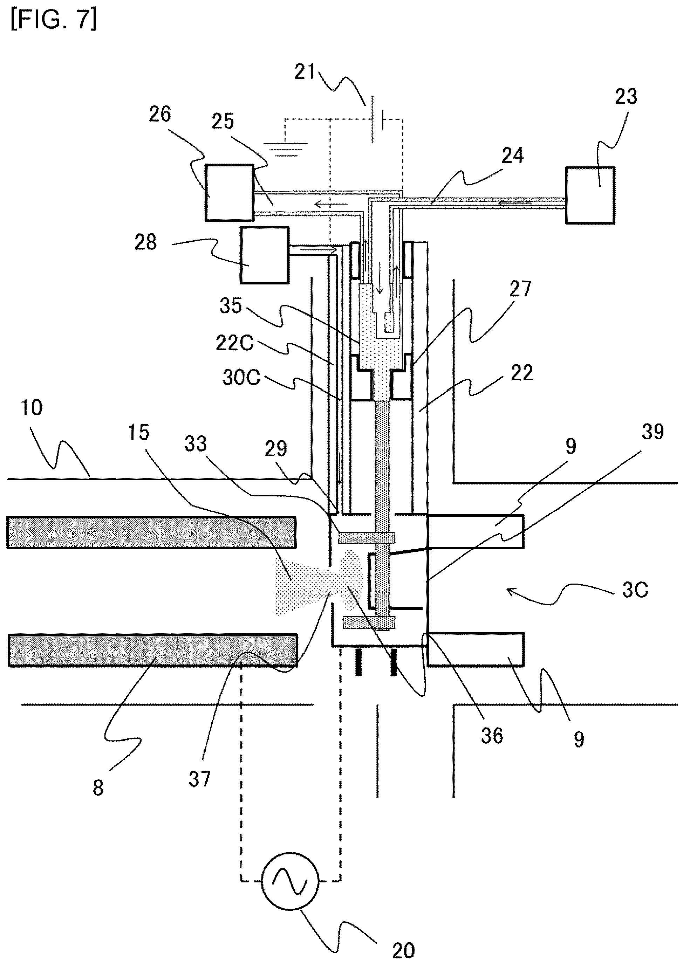

FIG. 7 illustrates an ion source 3C according to a third modified example. The ion source 3C illustrated in FIG. 7 is different from the ion source 3 illustrated in FIG. 4 in that an insulating gas tube 30C having a small diameter is disposed inside a gas supply flow path 22C, and a gas is supplied from a side of an extraction hole 37 to an electrical discharge chamber 36.

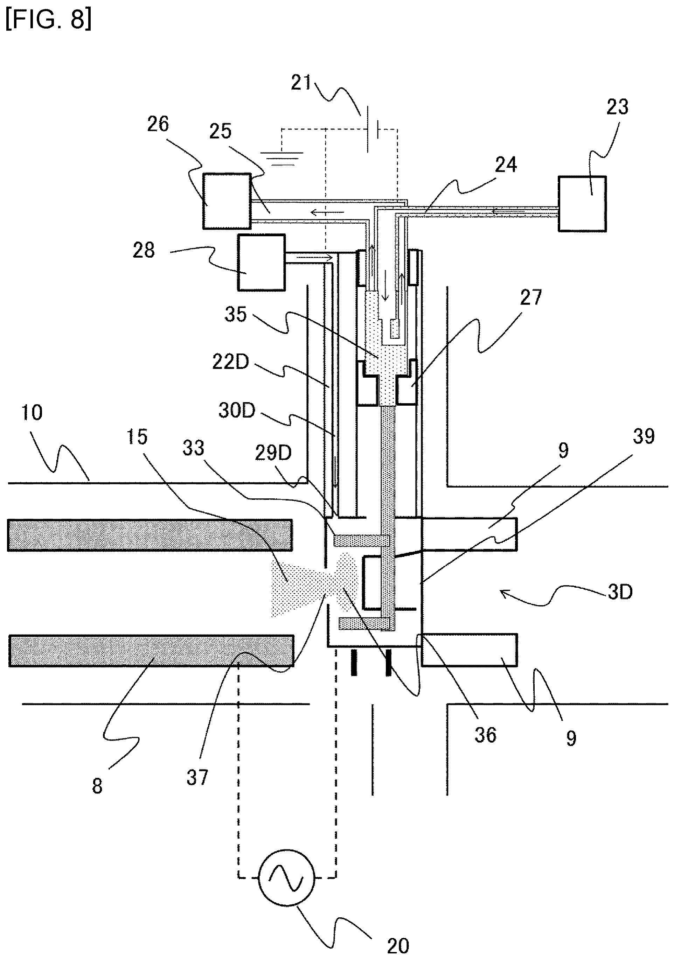

FIG. 8 illustrates an ion source 3D according to a fourth modified example. The ion source 3D illustrated in FIG. 8 is different from the ion source 3 illustrated in FIG. 4 in that, similarly to the ion source 3A illustrated in FIG. 5, central axes of cooling tubes 24 and 25 are eccentric with respect to a gas supply flow path 22D instead of being coaxial with the gas supply flow path 22D, an insulating gas tube 30D having a small diameter is disposed inside the gas supply flow path 22D similarly to the ion source 3C illustrated in FIG. 7, and a gas is supplied from a side of an extraction hole 37 to an electrical discharge chamber 36.

Next, effects of the present embodiment will be described.

Each of the ion sources 3, 3A, 3B, 3C, and 3D of the present embodiment described above includes the cathode 33, the cathode support tool 35 that supports the cathode 33 and applies and supplies a voltage, the cooling tubes 24 and 25 that cool the cathode support tool 35, and the gas supply flow path 22, 22A, 22B, 22C, or 22D that supplies the gas to the electrical discharge chamber 36, and the cooling tubes 24 and 25 are disposed on a flow-path inner peripheral side of the gas supply flow path 22, 22A, 22B, 22C, or 22D. With such a configuration, a cooling system and a raw material supply system can be more easily downsized, and downsizing of the ion source can be achieved as compared with a case where independent tubes are bundled.

In addition, as the gas supply flow path 22, 22A, 22B, 22C, or 22D and the cooling tubes 24 and 25 are integrated as the tubes having the triple structure, substantially the same structure as that in which independent tubes are disposed is obtained, so that downsizing can be more easily realized.

In addition, since the cooling tubes 24 and 25 and the gas supply flow path 22 or 22B have a common axis, it is possible to implement a simpler structure than a structure in which independent tubes are bundled, and thus, it is possible to more easily achieve downsizing.

Furthermore, since the central axes of the cooling tubes 24 and 25 are offset from the central axis of the gas supply flow path 22A or 22D, it is possible to prevent the gas from being diffused in the gas supply flow path 22A and not reaching the electrical discharge chamber 36 when a gas supply amount is small, and to appropriately supply the gas. In addition, since a cross-sectional area of the gas flow path is reduced, a size of the hole penetrating through the main magnetic pole 1 and the magnetic pole 10 can be reduced, so that the ion sources 3A and 3D can be further downsized.

In addition, since cross-sectional areas of the gas supply holes 29, 29A, and 29B, which are the outlets of the gas supply flow paths 22, 22A, and 22B, are smaller than cross-sectional areas of the gas supply flow paths 22, 22A, and 22B, it is possible to suppress the sample gas from being diffused and supplied to the electrical discharge chamber 36, and to avoid excessive gas supply.

Furthermore, since the gas supply hole 29, 29A, 29B, or 29D is disposed vertically above or below the extraction hole 37 through which the generated ions are emitted from the ion source 3, 3A, 3B, 3C, or 3D, the sample gas can be supplied in a region close to the electrical discharge chamber 36, and the sample gas can be ionized more efficiently.

In addition, since the gas supply flow paths 22C and 22D further include the gas tubes 30C and 30D provided in the outermost tubes, respectively, the gas is easily supplied to the electrical discharge chamber 36 even in a case where the amount of the gas is small.

Furthermore, in the cooling tubes 24 and 25, the coolant supply side is the inner peripheral side and the returning side is the outer peripheral side, and thus, tubes having a general configuration can be used, so that manufacturability can be improved.

In addition, the circular accelerator 50 including the ion source 3, 3A, 3B, 3C, or 3D as described above and the particle beam therapy system 100 including such a circular accelerator 50 can also be downsized by downsizing the ion sources 3, 3A, 3B, 3C, and 3D.

### <Others>

Note that the present invention is not limited to the above embodiments, and various modified examples and applications are possible. The above-described embodiments have been described in detail in order to explain the present invention in an easy-to-understand manner, and the present invention is not necessarily limited to those having all the configurations described.

For example, a type of the circular accelerator 50 is not limited to a cyclotron accelerator including the magnetic pole 10 with unevenness, and a synchrocyclotron accelerator that includes a magnetic pole inclined from the center of the magnetic pole 10 and modulates a frequency of radiofrequency acceleration can also be used.

The synchrocyclotron accelerator is a type of improved cyclotron accelerator, and applies a frequency-modulated radiofrequency electric field to charged particles that circularly move between large magnetic poles to repeatedly accelerate the charged particles. In addition, at the speed of light, a mass of the accelerated particles increases due to a relativistic effect, and a period of the circular motion of the charged particles in the magnetic field increases in proportion to the mass. A shift of the period from a radiofrequency voltage resulting therefrom is removed by modulating the frequency.

Since the synchrocyclotron accelerator has the same configuration as the cyclotron accelerator, the ion source 3, 3A, 3B, 3C, or 3D can be disposed in the main magnetic pole 1, and the same effects can be obtained.

The ion source of the present invention may have the following aspects.
(1) An ion source includes: a cathode; a cathode support tool that supports the cathode and applies and supplies a voltage; a cooling flow path that cools the cathode support tool; and a gas supply flow path that supplies a gas to an electrical discharge chamber, in which the cooling flow path is disposed on a flow-path inner peripheral side of the gas supply flow path.
(2) In the ion source according to (1), the cooling flow path and the gas supply flow path are integrated as tubes having a triple structure.
(3) In the ion source according to (1) or (2), the cooling flow path and the gas supply flow path have a common axis.
(4) In the ion source according to (1) or (2), a central axis of the cooling flow path is offset from a central axis of the gas supply flow path.
(5) In the ion source according to any one of (1) to (4), a cross-sectional area of a gas supply hole as an outlet of the gas supply flow path is smaller than a cross-sectional area of the gas supply flow path.
(6) In the ion source according to any one of (1) to (5), a gas supply hole as an outlet of the gas supply flow path is disposed vertically above or below an extraction hole through which generated ions are emitted from the ion source.
(7) In the ion source according to any one of (2) to (6), the gas supply flow path includes a tube further provided in an outermost tube.
(8) In the ion source according to any one of (1) to (7), in the cooling flow path, a coolant supply side is an inner peripheral side, and a returning side is an outer peripheral side.

### Reference Signs List

1 main magnetic pole
2 annular coil
3, 3A, 3B, 3C, 3D ion source
6 vacuum vessel
7 acceleration gap
8 radiofrequency acceleration electrode
9 ground electrode
10 magnetic pole
12 closed orbit
15 emitted beam
17 current monitor
20 radiofrequency power supply
21 electrical discharge power supply
22, 22A, 22B, 22C, 22D gas supply flow path
23 cooling material supply source
24, 25 cooling tube (cooling flow path)
26 cooling material returning unit
27 insulating spacer
28 gas supply source
29, 29A, 29B, 29D gas supply hole
30C, 30D gas tube
33 cathode
35 cathode support tool
36 electrical discharge chamber
37 extraction hole
39 electrical discharge vessel
40 treatment couch
45 patient
50 circular accelerator
52 beam transport line
54 irradiation nozzle
56 control apparatus
100 particle beam therapy system

## Claims

1. An ion source comprising:
a cathode;
a cathode support tool that supports the cathode and applies and supplies a voltage;
a cooling flow path that cools the cathode support tool; and
a gas supply flow path that supplies a gas to an electrical discharge chamber,
wherein the cooling flow path is disposed on a flow-path inner peripheral side of the gas supply flow path.

2. The ion source according to claim 1, wherein the cooling flow path and the gas supply flow path are integrated as tubes having a triple structure.

3. The ion source according to claim 2, wherein the cooling flow path and the gas supply flow path have a common axis.

4. The ion source according to claim 2, wherein a central axis of the cooling flow path is offset from a central axis of the gas supply flow path.

5. The ion source according to claim 1, wherein a cross-sectional area of a gas supply hole as an outlet of the gas supply flow path is smaller than a cross-sectional area of the gas supply flow path.

6. The ion source according to claim 1, wherein a gas supply hole as an outlet of the gas supply flow path is disposed vertically above or below an extraction hole through which generated ions are emitted from the ion source.

7. The ion source according to claim 2, wherein the gas supply flow path further includes a tube provided in an outermost tube.

8. The ion source according to claim 1, wherein in the cooling flow path, a coolant supply side is an inner peripheral side, and a returning side is an outer peripheral side.

9. A circular accelerator comprising:
magnetic poles facing each other;
an acceleration electrode; and
the ion source according to any one of claims 1 to 8 disposed between the facing magnetic poles and disposed parallel to a central axis of a circulating ion beam.

10. A particle beam therapy system comprising:
the circular accelerator according to claim 9;
a beam transport line;
an irradiation nozzle; and
a treatment couch.
